(19) Europäisches Patentamt
European Patent Office
Office européen des brevets

(11) EP 0 479 489 B1

(12) **EUROPEAN PATENT SPECIFICATION**

(45) Date of publication and mention of the grant of the patent:
**19.03.2003 Bulletin 2003/12**

(51) Int Cl.$^7$: **C07K 5/08**, A61K 38/06, A61K 38/55

(21) Application number: **91308785.4**

(22) Date of filing: **26.09.1991**

(54) **Tripeptide antithrombotic agents**

Antithrombotische Tripeptid-Wirkstoffe

Agents antithrombotiques à base de tripeptides

(84) Designated Contracting States:
**AT BE CH DE DK ES FR GB GR IT LI LU NL SE**

(30) Priority: **28.09.1990 US 589553**
**06.09.1991 US 756091**

(43) Date of publication of application:
**08.04.1992 Bulletin 1992/15**

(60) Divisional application:
**95201959.4 / 0 684 258**
**95201960.2 / 0 685 489**
**97203616.4 / 0 837 071**

(73) Proprietor: **ELI LILLY AND COMPANY**
**Indianapolis, Indiana 46285 (US)**

(72) Inventors:
- **Gesellchen, Paul David**
**Indianapolis, Indiana 46240 (US)**
- **Shuman, Robert Theodore**
**Greenwood, Indiana 46142 (US)**

(74) Representative: **Pritchard, Judith et al**
**Eli Lilly and Company Limited**
**Lilly Research Centre**
**Erl Wood Manor**
**Windlesham, Surrey GU20 6PH (GB)**

(56) References cited:
**EP-A- 0 185 390** **EP-A- 0 367 713**
**DE-A- 3 827 415**

- **Biochem Biophys Res Commun 101 (1981) 440-6; Peptides: Chemistry and Biol., Eds. Smith & Rivier, Proc of Am Pept Symp, June 16-21, 1991, 801-802.**

Remarks:
Divisional application 95201959.4 filed on 26/09/91.

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 479 489 B1

## Description

This invention relates to thrombin inhibitors which are useful anticoagulants in humans and animals. In particular it relates to derivatives of the dipeptide L-Proline-L-Arginine aldehyde having high antithrombotic activity.

Thrombin inhibition is currently achieved by the administration of heparins and coumarins. The mechanism by which these agents act has been much studied. Heparins are only administerable parenterally and levels must be carefully monitored. Coumarins act by blocking or inhibiting the formation of prothrombin and require some time to achieve maximum effectiveness.

Although both the heparins and the coumarins are effective anticoagulants there exists a need for antithrombin agents which act quickly to prevent clot formation and which do not interfere with plasmin action in dissolving existing clots.

Okamoto et al., BORC, 101, 440-446 (1981), discloses inhibition of thrombin by arginine derivative 805, a tetrahydroquinoline containing compound.

Shuman et al, Peptides: Chemistry and Biology, Eds. Smith and Rivier, Proceedings of the American Peptide Symposium, June 16-21, 1991, 801-802, is directed to tripeptide aldehydes which exhibit inhibition of thrombin.

The thrombin inhibiting compounds provided by this invention are represented by the following formula 1.

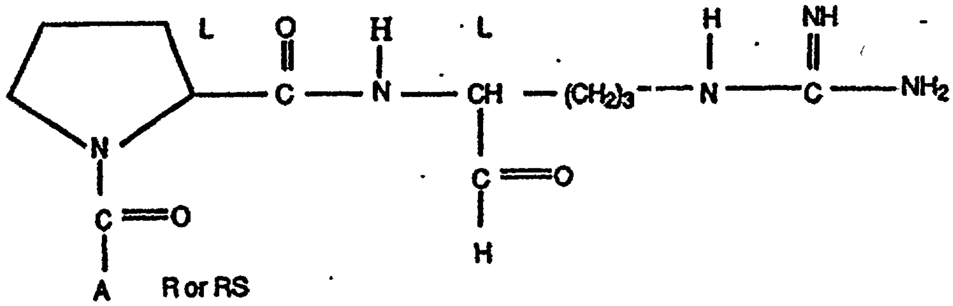

1

wherein A is a bicyclic group of the formula 2

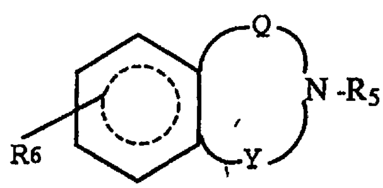

2

wherein Q is a one carbon radical represented by $-CH_2-$, and

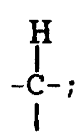

or a two carbon radical represented by $-CH_2-CH_2-$or

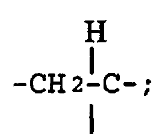

Y is a one carbon radical represented by -CH2-,

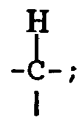

provided that when Q is

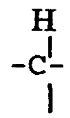 or 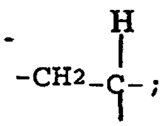

Y is $-CH_2-$, and when Y is

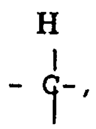

Q is $-CH_2-$ or $-CH_2-CH_2-$;

$R_5$ is hydrogen or an oxycarbonyl group of the formula $R^4$-OC(O)-wherein $R^4$ is $C_1$-$C_6$ alkyl, $C_2$-$C_6$ alkenyl, $C_3$-$C_7$ cycloalkyl, benzyl, nitrobenzyl, diphenylmethyl, or a phenyl group of the formula

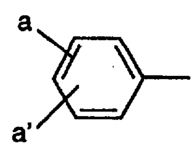

wherein a and a' independently are hydrogen, lower alkyl or lower alkoxy; halogen, trifluoromethyl, hydroxy, hydromethyl, amino, or aminomethyl; and $R_6$ is hydrogen, halogen, hydroxy, lower alkyl or lower alkoxy; and the dotted circle within the 6-membered ring indicates that the ring is an aromatic ring; and the pharmaceutically acceptable non-toxic salts thereof.

The peptides represented by the formula 1 are useful antithrombotic agents and can be used as adjuncts to tissue plasminogen activator (tPA), streptokinase or wokinase therapy.

The compounds are prepared by conventional coupling methods. For example, Boc-D-Phg is coupled with an ester of L-proline to form Boc-D-Phg-Pro ester. The ester group is removed and the Boc-D-Phg-Pro is coupled with the lactam form of L-arginine to provide Boc-D-Phg-Pro-Arg lactam in amino protected form. The Arg lactam ring is opened by reduction and the arginine amino protecting group removed to provide Boc-D-Phg-Pro-Arg aldehyde. The peptides are converted to suitable salt forms such as the acetates and sulfates.

As shown in formula 1, the asymmetric center of the A(C=0) moiety is R or RS while that of the proline and arginine aldehyde moieties is L.

The terms used in formula 1 are defined herein as follows:

Lower alkyl refers to the straight and branched chain $C_1$-$C_4$ alkyl groups such as methyl, ethyl, n-propyl, isopropyl, n-butyl and the like.

Lower alkoxy refers to $C_1$-$C_4$ alkoxy group such as methoxy, ethoxy, n-propoxy, isopropoxy, n-butoxy, t-butoxy and the like.

Halogen refers to fluoro, chloro, bromo or iodo.

Mono- or di-(lower alkyl)amino refers to such groups as methylamino, ethylamino, dimethylamino, methylethylamino, diethylamino, n-butylamino, n-propylamino and the like.

The term "$C_1$-$C_6$ alkyl" refers to the straight and branched alkyl groups such as the $C_1$-$C_4$ alkyl groups defined above and, in addition, n-pentyl, isopentyl, n-hexyl, the isomeric hexyl groups, and the like. "$C_2$-$C_6$ alkenyl" refers to the olefinic groups such as vinyl, allyl, butenyl, isomeric pentenyl and hexenyl groups. "$C_3$-$C_7$ Cycloalkyl" refers to the cyclic hydrocarbons having from three to 7 ring carbon atoms such as cyclopropyl, cyclobutyl, cyclopentyl, cyclohexyl

and cycloheptyl.

Examples of peptides represented by the formula 1 wherein A is a bicyclic group represented by the foregoing formula 2 are the D-1,2,3,4-tetrahydroisoquinolin-1-ylcarbonyl and D-1,2,3,4-tetrahydroisoquinolin-3-ylcarbonyl N-acyl derivatives of Pro-Arg-H depicted below,

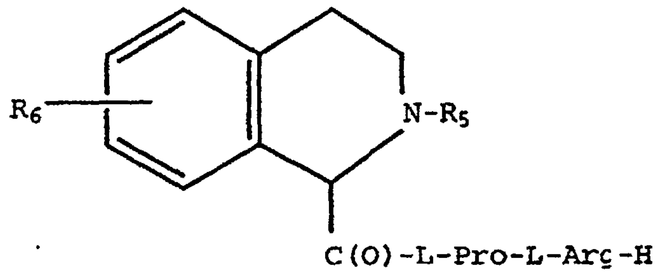

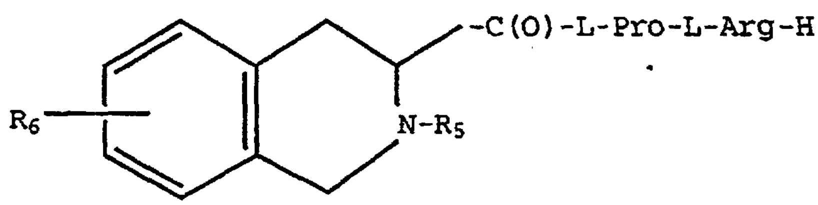

the dihydroisoindole-1-ylcarbonyl derivatives depicted below;

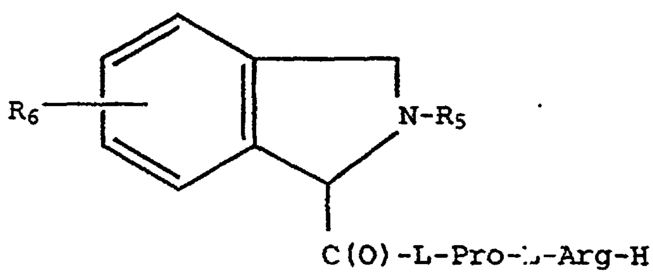

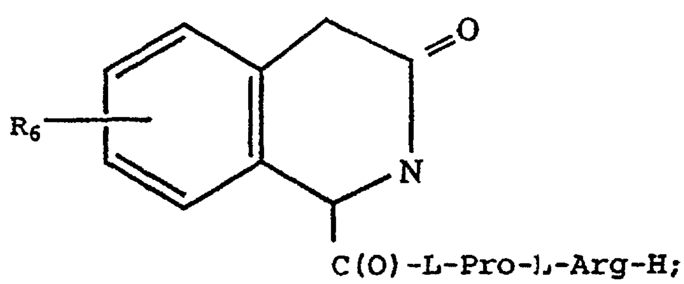

The terms $R_6$ and $R_5$ have the same meanings as defined hereinabove. $R_5$ is preferably hydrogen, and $R_6$ is preferably hydrogen, methoxy, ethoxy, chloro, or methyl.

Pharmaceutically acceptable salts of peptides of the invention include the acid addition salts formed with inorganic acids and carboxylic acids. Examples of inorganic acids forming salts are the hydrohalic acids hydrochloric and hydrobromic; phosphoric acid and sulfuric acid. Carboxylic acid salts are formed with acids such as acetic, propionic, malonic, maleic, citric, succinic, malic, benzoic, fumaric, and like carboxylic acids. The acid addition salts are prepared in a conventional manner e.g. by neutralizing the free base form of the compound 1 with the acid. Preferred acid addition salts are sulfate and hydrochloride salts.

A preferred embodiment of the invention comprises compounds of the formula 1 wherein A is a bicyclic group. Preferred compounds of this embodiment are represented by the formula 1 when A(C=O) is the 1,2,3,4-tetrahydroisoquinolin-1-ylcarbonyl (formula 2, Q = $-CH_2-CH_2-$,

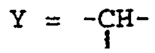

and, $R_5=R_6=H$) and 1,2,3,4-tetrahydroisoquinolin-3-ylcarbonyl (formula 2,

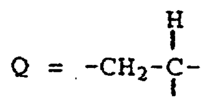

and Y = $-CH_2-$).

The compounds represented by the formula 1 are prepared by known methods of peptide coupling. According to one such method the acid A-COOH, wherein A has the same meanings as defined for formula 1, is coupled with a carboxy protected proline to form the dipeptide (when A is an amino acid) or an N-acylproline ester (when A is other than an amino acid). The carboxy protecting ester group of the proline moiety of the product is removed and the free acid form of the dipeptide is coupled with the lactam form of arginine. The above reaction sequence is illustrated by the following scheme

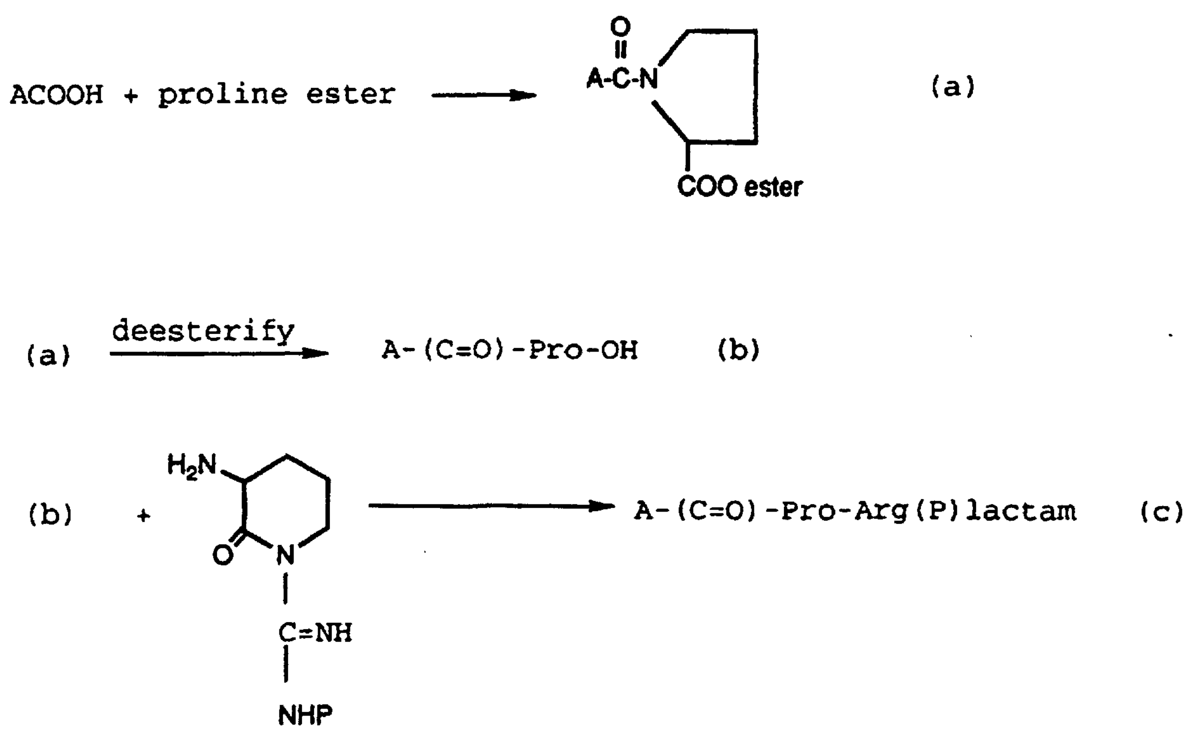


wherein P represents an amino protecting group.

The coupled Arg(P) lactam product (c) is reduced with lithium aluminum hydride in an inert solvent to cleave the lactam ring and provide the tripeptide in the arginine aldehyde form represented by the formula

A(C=O)-Pro-Arg(P)-H

wherein Arg(P)-H represents amino protected arginine aldehyde.

The lactam form of arginine is obtained by intramolecular coupling of amino protected arginine [Arg-OH]. For example, Boc-Arg(Cbz)OH represented by the formula

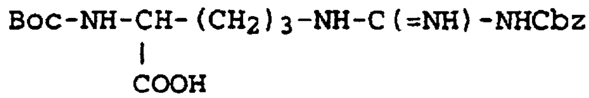

is first converted to an active ester form, such as an active mixed anhydride, with a chloroformate ester, e.g. ethyl chloroformate to isobutyl chloroformate. The ester formation is carried out in the presence of a tertiary amine such as N-methylmorpholine. Addition of a stronger tertiary amine base such as triethylamine effects the internal acylation to provide the lactam form of the di-amino protected arginine as shown below.

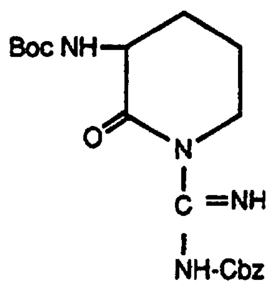

Prior to use in the coupling with the A(C=O)-Pro-OH as shown in the above scheme, the Boc protecting group is selectively removed with trifluoroacetic acid to provide the requisite free amino group.

The coupling of an ACOOH compound with a proline ester, when A is an amino acid residue, is carried out by first protecting the amino group of the amino acid. Conventional amino protecting groups commonly used for temporary protection or blocking of the amino group are employed. Examples of such protecting groups include the alkoxy, alkenyloxy, cycloalkoxy and aryloxycarbonyl groups such as ethoxycarbonyl, t-butyloxycarbonyl, cyclohexyloxycarbonyl, adamantyloxycarbonyl, trichloroethoxycarbonyl, benzyloxycarbonyl, diphenylmethoxycarbonyl, and like groups. The ester group employed to protect the carboxy group of proline during the coupling reaction can be any of the commonly used readily removable ester groups such as t-butyl, benzyl, p-nitrobenzyl, p-methoxybenzyl, diphenylmethyl, trichloroethyl, phenacyl, or trialkylsilyl esters. In carrying out the coupling reaction one employs an ester group for proline which is removable by conditions under which the amino protecting group remains intact. The amino protecting group of the acylating acid ACOOH thus remains in place for protection of the amino group during the subsequent coupling with the arginine lactam compound to form c.

The compounds of the formula 1 wherein A is a bicyclo group (2) are prepared by the same coupling methods as above. For example the peptide of formula 1 wherein A represents the 1,2,3,4-tetrahydroisoquinolin-1-yl group is obtained by acylation of an ester of proline, such as the benzyl ester, with an active derivative of 1,2,3,4-tetrahydro-1-carboxyisoquinoline. Active derivatives that can be used include the acid halides such as the chloride or bromide, the acid azide, as well as active esters and anhydrides such as those formed with the chloroformates as described above. The ring nitrogen of the tetrahydroisoquinoline (formula 2, $R_5$=H) is protected or alkylated during the acylative coupling. For example an active ester of N-Boc-1,2,3-4-t.etrahydro-1-carboxy-isoquinoline formed with iso-butyl chloroformate is used in the acylation of the proline ester. The peptide product N-Boc-1,2,3,4-tetrahydroisoquinolin-1-ylcarbonyl-proline ester is deesterified, the free acid converted to an active ester and the latter coupled to the lactam form of arginine. The lactam product is then converted to the aldehyde form as described above to provide the compound of the formula 1 namely, Boc-1,2,3,4-tetrahydroisoquinolin-1-ylcarbonyl-Pro-Arg-H.

The coupling reactions described above are carried out in the cold preferably at a temperature between about -20° C and about 15° C. The coupling reactions are carried out in an inert organic solvent such as dimethylformamide, dimethylacetamide, tetrahydrofuran, methylene chloride, chloroform, and like common solvents. Generally anhydrous conditions are used when, in the coupling reaction, an active ester of the acylating acid is used.

The compounds of the invention are isolated best in the form of acid addition salts. Salts of the compounds of formula 1 formed with acids such as those mentioned hereinabove useful as pharmaceutically acceptable salts for administration of the antithrombotic agents and for preparation of formulations of these agents. Other acid addition salts may be prepared and used in the isolation and purification of the peptides. For example, the salts formed with the sulfonic acids such as methanesulfonic acid, n-butanesulfonic acid, p-toluenesulfonic acid and naphthalene sulfonic acid may be so used.

The preferred method for isolating and purifying the compounds represented by the formula 1 while at the same time preparing a desired stable salt form is as follows. Stable salts of inorganic acids such as the sulfate and hydrochloride salts are provided by preparative purification over $C_{18}$ reversed-phase chromatography. The aqueous phase comprises sulfuric acid or hydrochloric acid at a concentration between about 0.01% and about 0.05% and acetonitrile, THF, methanol or other suitable solvent serves as the organic component. The pH of the acidic eluant is adjusted to between about pH 4 and about pH 6, the exact pH being a function of the particular peptide, with a basic resin e.g. Bio-Rad AG-1X8 resin in the hydroxyl form. After pH adjustment the solution of the tripeptide salt e.g. sulfate or hydrochloride, is lyophilized to provide the purified salt dry powder form. In an example of the process crude D-Phg-L-Pro-L-Arg-H sulfate, contaminated with the epimeric D-Arg-H sulfate is dissolved in ca 0.01% sulfuric acid and the solution is loaded on a Vydac $C_{18}$ RP-HPLC column. A gradient of 2-10% acetonitrile in 0.01% $H_2SO_4$ was used to elute the column over 10 hours. Multiple fractions are collected and those containing the desired product as determined by analytical RP-HPLC are pooled. The pH of the pooled fractions is adjusted to about pH 4.0 to about 4.5 with the Bio-Rad AG-1X8 resin in the hydroxyl cycle. After filtering the solution is lyophilized to provide pure D-Phg-L-Pro-L-Arg-H sulfate.

The compounds provided by the invention (formula 1) inhibit the action of thrombin in man and animals. The inhibition of thrombin is demonstrated by in vitro inhibition of amidase activity of thrombin. The following Table 1 lists the apparent equilibrium constant (Kass) for interaction between the test compound (inhibitor) and thrombin. The data in the table were obtained in an assay in which thrombin hydrolyzes the chromogenic substrate, N-benzoyl-D-phenylalanyl-L-valyl-L-arginyl-p-nitroanilide.

The assay was carried out in 50 µl buffer (0.03M Tris, 0.15M NaCl, pH 7.4) with 25 µl of thrombin solution (0.21 mg/ml of thrombostat powder in 0.06 M Tris, 0.3M NaCl, pH 7.4) and 150 µl of an aqueous solution of the chromogenic substrate at a concentration of 0.25 mg/ml. Solutions of test compound (25 µl) at various concentrations were added. Rates of hydrolysis of the substrate were measured by monitoring the reactions at 405 nm for the release of p-nitroaniline. Standard curves were constructed by plotting free thrombin concentration against hydrolysis rate. The hydrolysis rates observed with test compounds are then converted to "free thrombin" values in the respective assays by use of the standard curves. The bound thrombin (bound to test compound) was calculated by subtracting the amount of free thrombin observed in each assay from the known initial amount of thrombin used in the assay. The amount of free inhibitor in each assay was calculated by subtracting the number of moles of bound thrombin from the number of moles of added inhibitor (test compound).

The Kass value is the hypothetical equilibrium constant for the reaction between thrombin and the test compouhnd (I).

$$\text{Thrombin} + \text{I} \rightleftharpoons \text{Thrombin - I}$$

$$\text{Kass} = \frac{[\text{Thrombin I}]}{[(\text{Thrombin}) \times (\text{I})]}$$

Kass was calculated for a range of concentrations of test compounds and the mean value is reported in units of liter per mole.

TABLE 1

| Inhibition of Thrombin Amidase Activity | |
|---|---|
| Test Inhibitor[1] A(C=O)- | Thrombin Amidase Kass X$10^6$ (1/mole) |
| N-Boc-DL-1,2,3,4-tetrahydroisoquinolin-1-ylcarbonyl | 3.8 |
| DL-1,2,3,4-Tetrahydroisoquinolin-1-ylcarbonyl | 87.7 |

[1]/ A(C=O)- refers to formula 1. Unless indicated otherwise the test inhibitors were in the form of acetate salts.

The antithrombotic activity of representative compounds of the invention was determined in in vivo tests carried out in the rat. The test employed induced arterial thrombosis in the carotid artery of the rat and measured the infusion dose of test compound required to maintain blood flow for fifty minutes past the time of occlusion. The test was performed as follows.

Arterial thrombosis was induced in the rat by injury of the carotid artery. Topical application of a ferric chloride solution was used to injure the vessel. Male Sprague-Dawley rats (375-450 g) were anesthetized with xylazine (20 mg/kg, s.c.) followed by ketamine HCl (100 mg/kg, s.c.). Animals were laid on a water blanket in which the circulated water was maintained at 37° C. The carotid artery was approached through a midline cervical incision. Careful blunt dissection was used to expose and isolate the vessel from the carotid sheath. A silk suture was pulled underneath the artery to lift the vessel to provide clearance to insert a thermocouple underneath it. Vessel temperature changes were monitored on a strip chart recorder that had an ink-writing timer. Small forceps were used to dip discs (3 mm dia) of Whatman No. 1 filter paper into a $FeCl_3$ solution (35%). The discs were cut to equal size using sharpened stainless steel tubing (3 mm i.d.) chucked in a drill press. A saturated disc was placed on each carotid artery above the thermocouple. The time between $FeCl_3$ application and the time at which temperature decreased abruptly was recorded as time to occlusion (TTO) of the vessel. The average time required for both vessels to occlude was used to represent TTO for each animal.

Test compounds were dissolved in isotonic saline. A syringe pump was used to infuse drug solutions starting 15 min before $FeCl_3$ application and continuing for 60 min after $FeCl_3$ application. Dose-response curves were plotted to determine the relationship between the $\log_{10}$ of the infused doses and the TTO of the injured arteries. A comparative index of antithrombotic activity was determined from the curves by calculating the infusion dose required to maintain blood flow for 50 min (ED 50 min).

The association between vessel occlusion and the abrupt temperature decrease was established by simultaneously recording the temperature and blood flow on the same recorder. A pulsed Doppler flow probe was placed around the carotid artery proximal to the thermocouple. The probe recorded changes in flow velocity; therefore, it was installed at a point where thrombosis did not occur and the internal diameter of the vessel remained constant due to distention with fluid blood. Baseline temperature and flow velocity (determined with a Directional Pulsed Doppler Flowmeter, Model 545-C, University of Iowa Bioengineering) were recorded before application of 35% ferric chloride. Results were reported as percent change from initial baseline values (6 min before occlusion). The time at which vessel temperature decreased rapidly was arbitrarily established as zero and pre- and post-occlusion temperature and flow values were referenced from that point.

The compounds of the invention inhibit clot formation without appreciable interference with the bodies natural clot lysing ability e.g.the compounds have a low inhibitory effect on fibrinolysis.

The invention in one of its aspects provides a method for inhibiting the formation of blood clots in man and animals which comprises administering to said man or animal an effective clot inhibiting non-toxic dose of a compound represented by the formula 1. The anti-coagulant compound is administered orally, parenterally e.g. by intravenous infusion (iv), intramuscular injection (im) or subcutaneously (sc). Preferably administration is carried out by iv infusion.

An effective clot inhibiting dose is between about 5 mg and about 1000 mg. The dose regime may vary e.g. for prophylactic use a single daily dose may be administered or multiple doses such as 3 or 5 times daily may be appropriate. In critical care situations a compound of the invention is administered by iv infusion at a rate between about 1 mg/kg/h and about 50 mg/kg/h and preferably between about 2.5 mg/kg/h and about 25 mg/kg/h.

The method of this invention also is practiced in conjunction with a clot lysing agent e.g. tissue plasminogen activator (tPA), modified tPA, streptokinase or'urokinase. In cases when clot formation has occurred and an artery or vein is blocked, either partially or totally, a clot lysing agent is usually employed. A compound of the invention can be administered along with the lysing agent or subsequent to its use to prevent the reoccurrence of clot formation.

In carrying out the method the use of a preferred compound of the invention is desirable. An especially preferred compound of the invention for use in the method is N-(D-1,2,3,4-tetrahydroisoquinolin-1-oyl)-2-prolyl-2-arginine aldehyde sulfate.

The invention also provides pharmaceutical formulations for use in the above described therapeutic method.

Pharmaceutical formulations of the invention comprise an effective clot inhibitory amount of a compound represented by the formula 1 and a pharmaceutically acceptable carrier. For oral administration the antithrombotic conpound is formulated in gelatin capsules or tablets which may contain excipients such as binders, lubricants, disintegration agents and the like. For parenteral administration the antithrombotic is formulated in a pharmaceutically acceptable diluent e. g. physiological saline (0.9%), 5% dextrose, Ringer's solution and the like.

The antithrombotic compound of the invention can be formulated in unit dosage formulations comprising a dose between about 1 mg and about 1000 mg. Preferably the compound is in the form of a pharmaceutically acceptable salt such as for example the sulfate salt, acetate salt or a phosphate salt.

A preferred formulation is a unit dosage form comprising between 5 mg and 50 mg of N-(D-1,2,3,4-tetrahydroisoquinolin-1-oyl)-L-prolyl-L-arginine aldehyde sulfate in sterile ampoules.

The following Examples are provided to further describe the invention and are not to be construed as limitations thereof.

The Rf values in the following examples were determined by silica gel thin layer chromatography using Kieselgel 60F-254 (Merck, Darmstadt) in the following solvent systems:

(A) chloroform-methanol-acetic acid, 135:15:1,
v:v:v
(B) ethyl acetate-acetic acid-absolute ethanol,
90:10:10, v:v:v
(C) chloroform-methanol-acetic acid,
90:30:5, v:v:v

The analytical HPLC methods used in the examples were as follows:

<u>Method 1</u>. Waters 600E using a Vydac $C_{18}$ reversed-phase column of 0.46 cm x 10 cm. The chromatogram was monitored on an LDC at 220 nM using a gradient of A = 0.01M ammonium acetate and B = acetonitrile.

<u>Method 2</u>. Pharmacia FPLC using a PepRPC measuring 0.5 cm x 5.0 cm. Monitoring was done on a Pharmacia UV-M at 214 nM using a gradient of either A = 0.01M ammonium acetate or B = acetonitrile.

The abbreviations used in the examples have the following meanings.

Amino acids: Arg = arginine, Pro = proline, Phg = phenylglycine
Boc = t-butyloxycarbonyl
Bzl = benzyl
Cbz = benzyloxycarbonyl
DCC = dicyclohexylcarbodiimide
DMF = dimethylformamide
DMSO = dimethylsulfoxide
FAB-MS = fast atom bombardment mass spectrum
FD-MS = field desorption mass spectrum
THF = tetrahydrofuran
TLC = thin layer chromatography

## <u>Preparation 1</u>

N-Boc-D-Phenylglycyl-L-Prolyl-L-Arginine aldehyde (Boc-D-Phg-Pro-Arg-H) hemisulfate

1) Boc-D-Phg-Pro-OBzl

A solution of Boc-D-phenylglycine (15.0 g, 59.7 mmole) and proline benzyl ester hydrochloride (14.43 g, 59.7 mmole) in 60 ml of DMF was cooled to 0° C and N,N-diisopropylethylamine (10.3 ml, 59.7 mmole) was added followed by 1-hydroxybenzotriazole hydrate (8.1 g, 59.7 mmole) and DCC (12.3 g, 59.7 mmole). The reaction mixture was stirred for 3 days at room temperature after which it was filtered and the filtrate was evaporated to an oil under vacuum. The oil was dissolved in 200 ml of ethyl acetate and 150 ml of water and after shaking the organic layer was separated, washed three times with 100 ml portions of 0.1N HCl, once with 150 ml of water, three times with 100 ml portion of 5% sodium bicarbonate and again with 150 ml of water. The washed organic solution was dried over $MgSO_4$ and then was evaporated under vacuum to provide 24.8 g of Boc-D-Phg-Pro-OBzl as a solid (95% of theory): TLC $R_f$ (A) 0.75; FAB-MS 439 (M+).

2) Boc-D-Phg-Pro-OH

The Boc-D-Phg-Pro-OBzl product obtained as described above (24.5 g, 55.7 mmole) was dissolved in 40 ml of DMF and 225 ml of isopropyl alcohol and 1.0 g of 5% Pd on carbon catalyst were added to the solution. Nitrogen was bubbled into the reaction mixture via a gas dispersion tube and then hydrogen was bubbled through for 16 hours followed by a nitrogen purge for 5 minutes. The catalyst was filtered through a hyflo filter pad and the filtrate was evaporated to dryness under vacuum to yield a solid residue. The residue was crystallized from diethyl ether containing a small amount of ethyl acetate. There were obtained 10.35 g of the deesterified product. Boc-D-Phg-Pro (2), 53% yield: TLC $R_f$
(A) 0.32; FAB-MS 349 (MH+);

$^1$H (DMSO-$d_6$), δ 1.35 (s, 9H), 1.71-2.10 (m 4H), 3.10 (m, 1H), 3.74 (M, 1H), 4.20 (m 1H), 5.45 (d, 1H), 7.09 (d, 1H), 7.25-7.40 (m, 5H), 12.50 (bs, 1H).

3) Boc-L-Arg(Cbz)-OH

N-Boc-arginine hydrochloride (Boc-Arg-OH·HCl). (82.1 g, 250 mmole) was dissolved in 240 ml of 5N NaOH in a 3-necked round bottom flask. The solution was cooled to -5° C and benzyl chloroformate (143 ml, 1.0 mole, 4 eq.) was added dropwise over 55 min while the pH of the mixture was maintained at 13.2-13.5 with 5N NaOH (250 ml). The reaction mixture was stirred for one hour at -5° C after addition of the chloroformate was completed. The reaction mixture was diluted with 100 ml of water and 500 ml of diethyl ether and the aqueous layer was separated and extracted twice with 40 ml portions of diethyl ether. The aqueous layer was acidified to pH 3.0 with 3N $H_2SO_4$ (560 ml) and extracted with 550 ml of ethyl acetate. The separated aqueous layer was extracted once with ethyl acetate and the extract combined with the previous ethyl acetate extract. The combined extracts were washed with water, dried over $MgSO_4$ and evaporated to dryness under vacuum. The residue was triturated with ether and the precipitated product was filtered and dried. There were obtained 66.1 g of (3) Boc-Arg(Cbz)-OH (65% of theory): TLC $R_f$ (C) 0.43; FD-MS 408 (M+).

$^1$H NMR ($CDCl_3$), δ 1.42 (s, 9H), 1.61-1.91 (m, 4H), 3.23-3.41 (m, 2H), 4.17 (d, 1H), 5.21 (s, 2H), 5.62 (d, 1H), 7.30-7.42 (m, 6H), 8.37 (m, 1H),

4) Boc-Arg(Cbz)-Lactam

A solution of Boc-Arg(Cbz)-OH (3) prepared as described above (66.0 g, 0.162 mole) in 230 ml of dry THF was cooled to -10° C in an ice-acetone bath. To the cold solution was added N-methylmorpholine (18.7 ml, 1.05 eq) followed by isobutyl chloroformate (22.5 ml, 1.05 eq) and the mixture was stirred for 5 minutes at -10° c. Next, triethylamine (23.5 ml, 1.05 eq) was added and the mixture was stirred for 1 h at -10° C and at room temperature for 1 h. The reaction mixture was poured into one liter of an ice-water mixture and the product (4) precipitated. The precipitate was filtered, washed with cold water, dried under vacuum, and was crystallized from ethyl acetate. There was obtained 38.05 a (60% of theory) of the product 4, Boc-Arg(Cbz)-lactam: TLC $R_f$ (A) 0.77; FD-MS 391 (MH+).

$^1$H NMR ($CDCl_3$) δ 1.48 (s, 9H), 1.78-1.98 (m, 2H), 2.50 (m, 1H), 3.41 (m, 1H), 4.43 (m, 1H), 4.90 (m, 1H), 4.16 (s, 2H), 5.27 (m, 1H), 7.28-7.45 (m, 6H), 9.41 (m, 1H), 9.68 (m, 1H).

5) Arg(Cbz)-Lactam Trifluoroacetate salt.

Boc-Arg(Cbz)-lactam (4) (38.0 g, 0.097 mole) was mixed with 200 ml of trifluoroacetic acid containing 20 ml of anisole and the mixture was stirred at 0° C for one hour. The reaction mixture was evaporated under vacuum without heating and 400 ml of diethyl ether were added to the residue. The resulting solid was filtered, washed with diethyl ether and dried under vacuum. There were obtained 40.5 g of the product (5) trifluoroacetate salt: TLC $R_f$ (C) 0.29; FD-MS 291 (MH+).

6) Boc-D-Phg-Pro-Arg(Cbz)-Lactam

To a solution of Boc-D-Phg-Pro (14.5 g, 41.6 mmole), prepared as described in part 2 above, in 80 ml of DMF cooled to -15° C was added 4.6 ml of N-methylmorpholine followed by 5.4 ml of isobutyl chloroformate and the reaction mixture was stirred at -15° C for two minutes.

In a separate flask TFA · Arg (Cbz) lactam (15.3 g, 37.8 mmole, prepared as described above in part 5), was dissolved in 30 ml of DMF, the solution cooled to 0° C and 4.6 ml of N-methylmorpholine were added. After the solution was stirred at 0° C for two minutes it was poured into the Boc-D-Phg-Pro solution prepared as described above. The reaction mixture obtained was stirred for 4 h at -15° C and then was allowed to warm to room temperature overnight.

A 5% solution of $NaHCO_3$ (5 ml) was added to the reaction mixture which was then evaporated under vacuum to provide an oil. The oil was dissolved in 175 ml of ethyl acetate and 150 ml of water were added. After shaking, the organic layer was separated, washed with 5% $NaHCO_3$ and water, dried over $MgSO_4$ and evaporated to dryness under vacuum to yield 23.0 g of Boc-D-Phg-Pro-Arg(Cbz) lactam (6) as an amorphous solid (98% yield). TLC $R_f$ (A) 0.72. FAB-MS 621 (MH+).

7) Boc-D-Phg-Pro-Arg(Cbz)-H

The lactam (6) (23.0 g, 37 mmole), obtained as described in part 6 above was dissolved in 200 ml of dry THF and the solution cooled to -15° C under a nitrogen atmosphere. To the cold solution was added dropwise over 10 min a 1M solution of lithium aluminum hydride in THF (37 ml, 37 mmole) and after addition was complete the reaction mixture was warmed to 0° C and stirred for 1 h. A solution of 12 ml of THF and 12 ml of 0.5N $H_2SO_4$ was slowly added dropwise to the mixture over 10 min. The reaction mixture was diluted with 200 ml of ethyl acetate and 200 ml of water and after shaking, the organic layer was separated. The organic layer was washed 3 times with 150 ml portions of water, dried over $MgSO_4$ and evaporated to dryness under vacuum to yield 19.2 g (83% yield) of Boc-D-Phg-Pro-Arg (Cbz)-H. FAB-MS 623 (MH+). $[\alpha]_D$ = -66.1°, C = 0.5, $CHCl_3$.

8) Boc-D-Phg-Pro-Arg-H hemisulfate

Boc-D-Phg-Pro-Arg(Cbz)-H (7) (18.2 g, 29.2 mmole) was dissolved in 100 ml of THF and 100 ml of water and 29.2 ml of

1N $H_2SO_4$ and 2 g of 10% Pd/C were added to the solution. Nitrogen was bubbled into the suspension for 5 min via a gas dispersion tube followed by hydrogen for 4 h. After the reduction was complete nitrogen was bubbled through again for 5 minutes. The reaction mixture was filtered through a hyflo pad to remove the catalyst and the filtrate was concentrated to a volume of 100 ml by evaporation under vacuum. To the aqueous concentrate were added 200 ml of n-butanol and the organic layer was separated from the aqueous layer. The aqueous was extracted three times with 100 ml portions of n-butanol and the extracts were combined and added to the organic layer. The organic layer was evaporated to dryness under vacuum, the residue triturated with diethyl ether/diisopropyl either, 1:1, v:v, and the solid filtered and dried under vacuum to yield 10.26 g of the crude product (8). The crude material was dissolved in 10% acetonitrile-water and the solution applied to a 7.5 cm x 53 cm column of HP-20 resin previously equilibrated in 10% acetonitrile-water. The product was eluted from the columns by step-wise elution with increasing concentrations of acetonitrile in water (10% to 12% to 15%). Multiple fractions were collected and assayed for the product via reversed-phase HPLC. Fractions containing the product were pooled and evaporated to dryness to yield 5.42 g of the pure product, Boc-D-Phg-Pro-Arg-H hemisulfate, (53% yield): $[\alpha]_D$ = -125.6°, C = 0.5 $CHCl_3$; FAB-MS 489 (MH+); Retention Time on RP-HPLC (Method 2, 10-50% B over 45 min., time = 32.3 min.

**Examples 1-3**

Examples 1 to 3 listed in the following Table 4 were prepared by the coupling methods described by Preparation 1 when the indicated amino acid or substituted acetic acid [A(C=O)] was substituted for the D-phenylglycine used in Example 1. All of the compounds in Table 4 are acetate salt forms.

**TABLE 2**
**A(C=O)-Pro-Arg-H**

| Ex. No. | A(C=O)- | MS[1] | HPLC Method[2] No./Gradient | Retention Time (min) |
|---|---|---|---|---|
| | N-Boc-1,2,3,4-tetrahydroisoquinolin-1-ylcarbonyl | 515 | 1/20% to 60% B over 60 min. | 49.0 |
| | D-1,2,3,4-Tetrahydroisoquinolin-1-ylcarbonyl | 415 | 2/5% to 50% B over 60 min. | 30.6 |
| | D-1,2,3,4-Tetrahydroisoquinolin-3-ylcarbonyl | 415 | 2/5% to 50% B over 60 min. | 26.4 |

1/ FAB-MS($MH^+$)

2/ The HPLC method is that described above

**Example 8**

D-1,2,3,4-Tetrahydroisoquinolin-1-oyl-L-Prolyl-L-Arginine aldehyde Sulfate

To a solution of isoquinoline-1-carbcxylic acid (12.5, 0.072 mole) in 185 ml of glacial acetic acid was added 2 g of platinum oxide and the suspension was hydrogenated at room temperature under 60 psi hydrogen pressure in a Parr hydrogenation apparatus for 24 h. The reaction mixture was filtered though a filter pad (Celite) to remove the catalyst and the filtrate was evaporated to dryness in vacuo. The solid residue was triturated with water, filtered and dried to yield 8 g (63% yield) of DL-1,2,3,4-tetrahydroisoquinolin-1-carboxylic acid. FD-Mass spectrum 178 ($MH^+$); $^1H$ NMR (DMSO-$d_6$): δ 2.80-3.00 (m, 3H), 3.15 (m, 1H), 3.30-3.40 (m, 2H), 7.05-7.25 (m, 4H), 7.70 (m, 1H).

The product (7.08 g, 0.04 mole) was dissolved in 2N NaOH (40 ml, 0.08 mole) and 40 ml of t-butyl alcohol and 10.5 g (0.048 mole) of di-tert-butyl dicarbonate were added to the solution. After stirring for 24 h at room temperature the bulk of the t-butyl alcohol was evaporated from the reaction mixture. The resulting aqueous solution was extracted with diethyl ether, the aqueous layer separated and acidified with 2N HCl to pH 2.0. The acidified aqueous phase was extracted with ethyl acetate, the extract dried over $MgSO_4$ and evaporated to dryness in vacuo. The residual oil was dissolved in diethyl ether and 7.9 ml (0.04 mole) of dicyclohexylamine was added to the solution. After standing at 4° C for 4 h the precipitate of the dicyclohexylamine salt of N-Boc-DL-1,2,3,4-tetrahydro-isoquinolin-1-carboxylic acid was filtered, washed with diethyl ether and dried in vacuo.There were obtained 15.7 g (86% yield) of the pure salt. FD-Mass spectrum 459 ($MH^+$).
Elemental analysis calculated for $C_{27}H_{42}N_2O_4$:

| | | | |
|---|---|---|---|
| Theory | C, 70.71; | H, 9.23; | N, 6.11 |
| Found | C, 71.07; | H, 9.37; | N, 5.87 |

The Boc protected derivative (73.4 g, 160 mmole) was suspended in 200 ml of ethyl acetate and the suspension was washed with 1.5 N citric acid and water, was dried over $MgSO_4$ and evaporated to dryness under vacuum. The residual oil was dissolved in ethyl acetate, the solution cooled to 0° C and 2,4,5-trichlorophenol (31.6 g, 160 mmole) was added to the solution followed by DCC (33 g, 160 mmole). The reaction mixture was stirred for one hour at 0° C and at room temperature for 1.5 h. The reaction mixture was cooled to 0° C the precipitate filtered and the filtrate evaporated to dryness under vacuum. The residual oil was dissolved in 100 ml of pyridine and proline (18.42 g, 160 mmole) and triethylamine (22.3 ml, 160 mmole) were added to the solution. After stirring at room temperature for 24 h. the reaction mixture was evaporated to dryness under vacuum. The residue was dissolved in ethyl acetate, water was added and the pH adjusted to 9.5 with 2N NaOH. The aqueous layer was separated, acidified to pH 2.0 with 2N HCl, and extracted with ethyl acetate. The extract was dried over $MgSO_4$, filtered and evaporated to dryness under vacuum. The oil residue was dissolved in methylene chloride and ethyl acetate. After standing at 4° C for 4 h a precipitate formed in the solution, was filtered, washed with ethyl acetate and recrystallized from methylene chloride/ethyl acetate. The solid product, Boc-D-1,2,3,4-tetrahydroisoquinolin-1-oyl-L-proline (Boc-D-1-Tiq-Pro-OH), was dried under vacuum to give 19.6 g, 33% yield of the pure product, TLC $R_f$ (A) 0.44; FAB-MS, 375 ($MH^+$);

Elemental analysis calculated for $C_{20}H_{26}N_2O_5$;

| | | | |
|---|---|---|---|
| Theory | C, 64.15; | H, 7.00; | N, 7.48 |
| Found | C, 63.26; | H, 6.98; | N, 7.52 |

$[\alpha]_D$ = +43.14°, C = 0.5, methanol.

In a first flask, Boc-D-1-Tiq-Pro (17.8 g, 47.5 mmole) was dissolved in 100 ml of DMF, the solution cooled to -15° C and 5.3 ml (52.3 mmole) of N-methylmorpholine and 6.2 ml (47.5 mmole) of isobutylchloroformate were added. The mixture was stirred at -15° C for two min.

In a second flask, the Cbz protected arginine lactam as the trifluoroacetate salt [Arg(Z)-Lactam·TFA], (19.2 g, 47.5 mmole) was dissolved in 40 ml of DMF, the solution cooled to 0° C, and 5.3 ml (52.3 mmole) of N-methylmorpholine were added. The mixture was stirred for 2 min at 0° C before being added to the first flask. The reaction mixture was stirred for 4 h at -15° C, then was slowly warmed to room temperature overnight and 5 ml of 5% NaHCO3 was added. The reaction mixture was evaporated under vacuum to provide an oil. The oil was dissolved in 175 ml of ethyl acetate and 150 ml of water were added to the solution. The organic layer was separated, washed with 5% $NaHCO_3$, water, 0.1N HCl and with water again before drying over $MgSO_4$. The washed and dried solution was evaporated under vacuum to dryness to yield 24.3 g (79% yield) of the product. Boc-D-1-Tiq-Pro-Arg(Z) lactam, as an amorphous solid.

TLC $R_f$ (A) 0.71
FAB-MS 647 ($MH^+$)

$[\alpha]_D$ = -32.8°, C = 0.5 chloroform

The Arg(Z) lactam product obtained above (23.4 g, 36.2 mmole) was dissolved in 300 ml of dry THF and the solution placed under $N_2$. The solution was cooled to -20° C and 37 ml lithium aluminum hydride 1M in THF (37 mmole) was added dropwise to the cold solution over 30 min. After addition was completed the mixture was stirred at -20° C for 30 min, and a solution of 20 ml of THF and 20 ml of 0.5N $H_2SO_4$ was added dropwise over 10 min. The reaction mixture was diluted with 400 ml of ethyl acetate and 400 ml of water were added. The organic layer was separated, washed twice with 150 ml portions of water and dried over $MgSO_4$. The washed and dried organic layer was evaporated under vacuum to yield 21 g (89% yield) of the product, Boc-D-1-Tiq-Pro-Arg(Z)-H, as an amorphous solid.

TLC $R_f$(A) 0.28.

The Arg(Z)-H product obtained as described aboved was hydrogenated as follows to remove the Cbz protecting group. The product (18.1 g, 27.9 mmole) was dissolved in 200 ml of THF and 80 ml of water and 28 ml of 1 N $H_2SO_4$ and 3.0 g of 5% Pd-on-carbon were addded. Nitrogen was bubbled through the suspension via a gas dispersion tube for 5 min. followed by hydrogen for 5 h and thereafter nitrogen for 5 min. The catalyst was filtered and the filtrate concentrated to a volume of 100 ml. The concentrate was diluted with 200 ml of n-butanol and the layers separated. The aqueous layer was extracted three times with 100 ml portions of n-butanol and the extracts were combined with the organic layer. The organic layer was evaporated under vacuum, and the reaction product residue triturated with diethyl ether:diisopropyl ether, 1:1, v:v, the solid was filtered and dried under vacuum to give 11.08 g of crude product.

The product was purified and obtained as the sulfate salt as follows. The crude product obtained as described above was dissolved in 20 ml of water and 20 ml of 10N $H_2SO_4$. The solution was heated at 50° C for 25 min., cooled to room temperature and the pH of the solution was adjusted to 4.0 with Bio-Rad AG1-X8 resin (hydroxide form). The resin was separated from the solution by filtration and the solution was lyophilized to yield 8.44 g of the crude product as the sulfate salt, D-1-Tiq-Pro-Arg-H•$H_2SO_4$.

The sulfate salt (4.2 g) was dissolved in 0.01% $H_2SO_4$ and the solution applied to two 5 cm x 25 cm HPLC reversed phase columns (Vydac $C_{18}$ resin) in series. A gradient of increasing concentrations of acetonitrile (2% to 10%) was used to elute the product salt. Fractions were collected and pooled on the basis of analytical RP-HPLC profile. The pH of the combined fractions was adjusted to 4.0 using AG1-X8 resin (Bio-Rad analytical anion exchange resin of 50-100 mesh) in the hydroxy cycle. The solution was filtered to remove the resin and the filtrate was lyophilized. There were obtained 2.4 g (57% of theory) of the purified product

FAB-MS 415 ($MH^+$)

$[\alpha]_D$ = -76.12°, C = 0.5/0.01N $H_2SO_4$

Amino acid analysis: Pro, 0.92; Tiq, 1.00

Elemental analysis calculated for $C_{21}H_{32}N_6O_7S$:

| | | | | |
|---|---|---|---|---|
| Theory | C, 49.21; | H, 6.29; | N, 16.29; | S, 6.26 |
| Found | C, 51.20; | H, 6.17; | N, 16.88; | S, 5.37. |

## Claims

### Claims for the following Contracting States : AT, BE, CH, DE, DK, FR, GB, IT, LI, LU, NL, SE

**1.** A compound of the formula

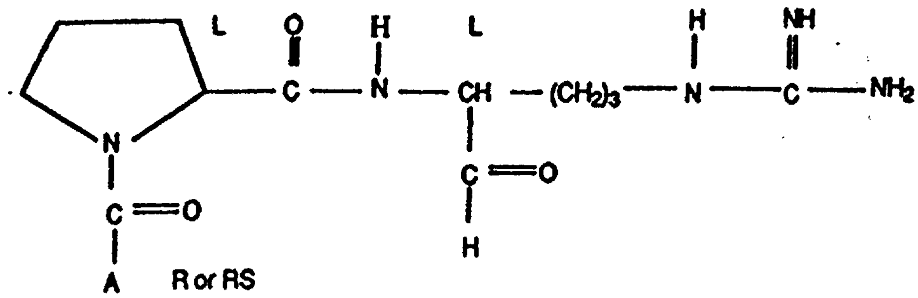

1

wherein A is a bicyclic group of the formula

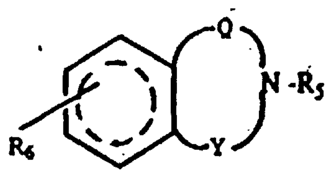 2

wherein Q is a one carbon radical represented by $-CH_2-$, and

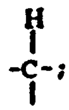

or a two carbon radical represented by $-CH_2-CH_2-$ or

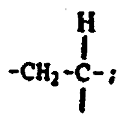

Y is a one carbon radical represented by $-CH_2-$ or

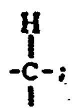

provided that when Q is

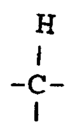 or 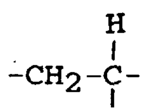 ,

Y is $-CH_2-$, and when Y is

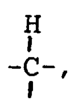

Q is $-CH_2-$ or $-CH_2-CH_2-$;
$R_5$ is hydrogen or an oxycarbonyl group of the formula

$$R_4\text{-OC(O)-}$$

wherein $R_4$ is $C_1$-$C_6$ alkyl, $C_2$-$C_6$ alkenyl, $C_3$-$C_7$ cycloalkyl, benzyl, nitrobenzyl, diphenylmethyl, or a phenyl group of the formula

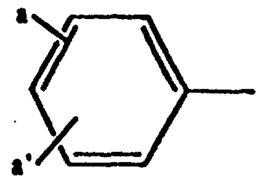

wherein a and a' independently are hydrogen, lower alkyl, lower alkoxy, halogen, trifluoromethyl, hydroxy, hydromethyl, amino, or aminomethyl; and
$R_6$ is hydrogen, halogen, hydroxy, lower alkyl or lower alkoxy; and the dotted circle within the 6-membered ring indicates that the ring is an aromatic ring;
and the pharmaceutically acceptable non-toxic salts thereof.

**2.** A compound of the formula

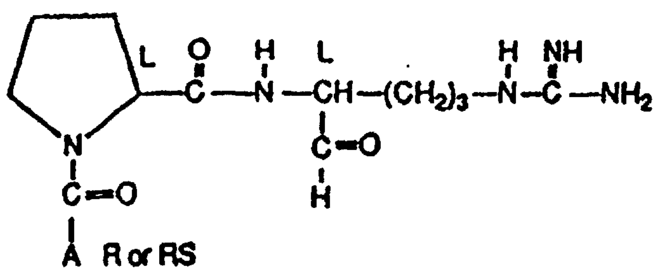

wherein A is a bicyclic group of the formula

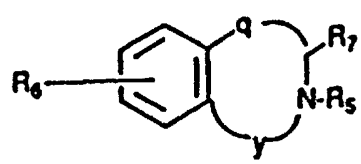

3

wherein q is a carbon to carbon bond or $-CH_2-$; Y is a carbon to nitrogen bond or $-CH(R_8)-$, and $R_7$ and $R_8$ are hydrogen or >C=O, provided that when $R_7$ is >C=O Y is a carbon to nitrogen bond, $-CH(R_8)$ and $R_4$ is hydrogen, and q is $-CH_2-$; $R_5$ is hydrogen, lower alkyl, benzyl or an oxycarbonyl group as defined in claim 1; and $R_6$ is hydrogen, halogen, hydroxy, lower alkyl or lower alkoxy; and the pharmaceutically acceptable non-toxic salts thereof

**3.** The compound of claim 1 said compound being DL-1,2,3,4-tetrahydroisoquinolin-1-oyl-L-prolyl-L-arginine aldehyde sulfate.

**4.** The compound of claim 1 said compound being D-1,2,3,4-tetrahydroisoquinolin-1-oyl-L-prolyl-L-arginine aldehyde sulfate.

**5.** The compound of claim 1 said compound being D-1,2,3,4-tetrahydroisoquinolin-3-oyl-L-prolyl-L-arginine aldehyde and pharmaceutically acceptable salts thereof.

**6.** A compound as claimed in any of claims 1-5 for use as an antithrombotic agent.

**7.** A pharmaceutical formulation comprising as an active ingredient a compound as claimed in any of claims 1-5, associated with one or more pharmaceutically acceptable carriers, excipients or diluents therefor.

**Claims for the following Contracting States : ES, GR**

**1.** A process for preparing a compound of the formula

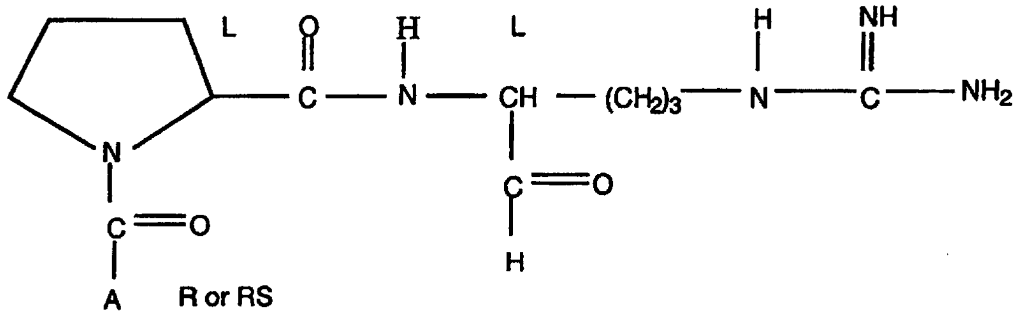

R or RS

1

wherein A is a bicyclic group of the formula

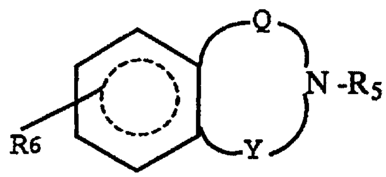

2

wherein Q is a one carbon radical represented by $-CH_2-$, and

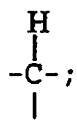

or a two carbon radical represented by $-CH_2-CH_2-$ or

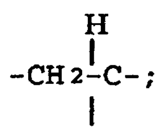

Y is a one carbon radical represented by -CH2-,

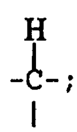

provided that when Q is

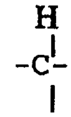 or 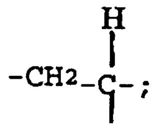

Y is $-CH_2-$, and when Y is

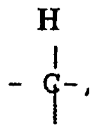

Q is $- CH_2-$ or $-CH_2-CH_2-$;
$R_5$ is hydrogen or an oxycarbonyl group of the formula $R^4$-OC(O)-wherein $R^4$ is $C_1$-$C_6$ alkyl, $C_2$-$C_6$ alkenyl, $C_3$-$C_7$ cycloalkyl, benzyl, nitrobenzyl, diphenylmethyl, or a phenyl group of the formula

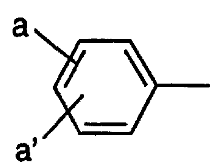

wherein a and a' independently are hydrogen, lower alkyl or lower alkoxy; halogen, trifluoromethyl, hydroxy, hydromethyl, amino, or aminomethyl; and $R_6$ is hydrogen, halogen, hydroxy, lower alkyl or lower alkoxy; and the dotted circle within the 6-membered ring indicates that the ring is an aromatic ring: and the pharmaceutically acceptable non-toxic salts thereof.

**2.** A process for preparing a compound of the formula

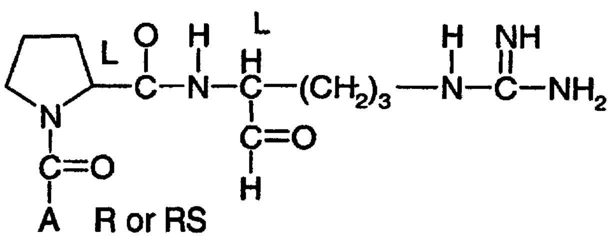

R or RS

wherein A is a bicyclic group of the formula

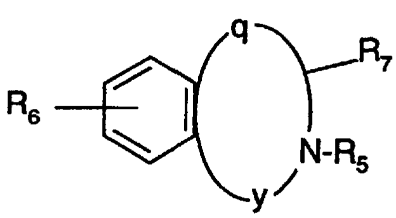 3

wherein q is a carbon to carbon bond or $-CH_2-$; Y is a carbon to nitrogen bond or $-CH(R_8)-$, and $R_7$ and $R_8$ are hydrogen or >C=O, provided that when $R_7$ is >C=O Y is a carbon to nitrogen bond, $-CH(R_8)$ and $R_8$ is hydrogen,

and q is $-CH_2-$; $R_5$ is hydrogen, lower alkyl, benzyl or an oxycarbonyl group as defined in claim 1; and $R_6$ is hydrogen, halogen, hydroxy, lower alkyl or lower alkoxy; and the pharmaceutically acceptable non-toxic salts thereof.

**3.** A process according to claim 1 for the preparation of DL-1,2,3,4-tetrahydroisoquinolin-1-oyl-L-prolyl-L-arginine aldehyde sulfate.

**4.** A process according to claim 1 for the preparation of D-1,2,3,4-tetrahydroisoquinolin-1-oyl-L-prolyl-L-arginine aldehyde sulfate.

**5.** A process according to claim 1 for the preparation of DL-1,2,3,4-tetrahydroisoquinolin-l-oyl-L-prolyl-L-arginine aldehyde sulfate and pharmaceutically acceptable salts thereof.

## Patentansprüche

**Patentansprüche für folgende Vertragsstaaten : AT, BE, CH, DE, DK, FR, GB, IT, LI, LU, NL, SE**

**1.** Verbindung der Formel

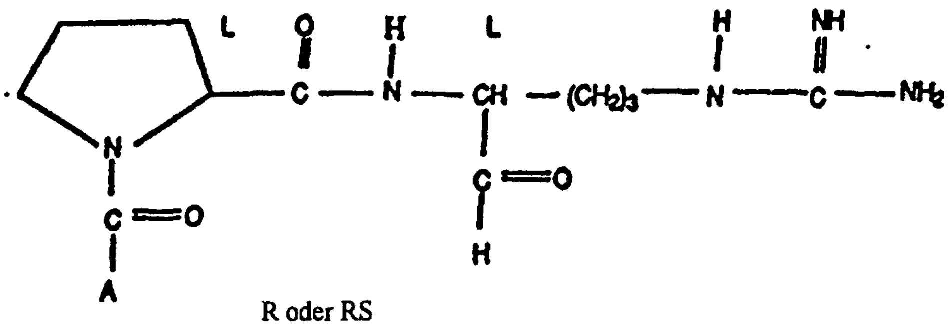

1

R oder RS

worin A für eine bicyclische Gruppe der folgenden Formel steht

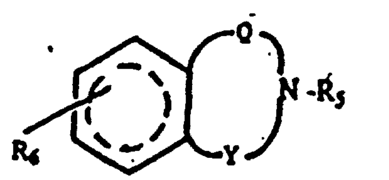

2

worin Q für einen Rest mit einem Kohlenstoff steht, der dargestellt wird durch $-CH_2-$ und

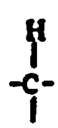

oder einen Rest mit zwei Kohlenstoffen, der dargestellt wird durch $-CH_2-CH_2-$ oder

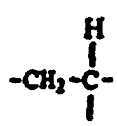

Y für einen Rest mit einem Kohlenstoff steht, der dargestellt wird durch $-CH_2-$ oder

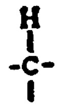

mit der Maßgabe, daß wenn Q für

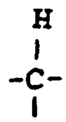

oder

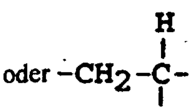

steht, Y dann für $-CH_2-$ steht, und wenn Y für

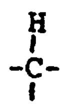

steht, Q dann fiir $-CH_2-$ oder $-CH_2-CH_2-$ steht.
$R_5$ für Wasserstoff oder eine Oxycarbonylgruppe der Formel $R_4-OC(O)-$ steht,
worin $R_4$ für $C_1$-$C_6$ Alkyl, $C_2$-$C_6$ Alkenyl, $C_3$-$C_7$ Cycloalkyl, Benzyl, Nitrobenzyl, Diphenylmethyl oder eine Phenylgruppe der folgenden Formel steht

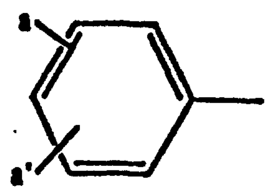

worin a und a' unabhängig für Wasserstoff, Niederalkyl, Niederalkoxy, Halogen, Trifluormethyl, Hydroxy, Hydromethyl, Amino oder Aminomethyl stehen und
$R_6$ für Wasserstoff, Halogen, Hydroxy, Niederalkyl oder Niederalkoxy steht und der gestrichelte Ring innerhalb des sechsgliedrigen Rings anzeigt, daß der Ring ein aromatischer Ring ist
und pharmazeutisch annehmbare, nicht-toxische Salze hiervon.

**2.** Verbindung der Formel

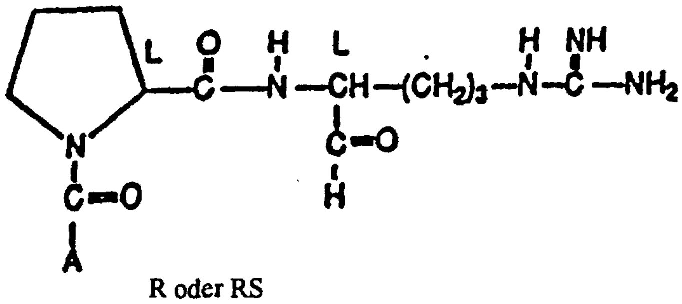

R oder RS

worin A für eine bicyclische Gruppe der folgenden Formel steht

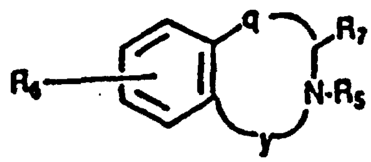

3

worin q für eine Kohlenstoff-Kohlenstoff-Bindung oder $-CH_2-$ steht,
Y für eine Kohlenstoff-Stickstoff-Bindung oder $-CH(R_8)-$ steht und
$R_7$ und $R_8$ für Wasserstoff oder >C=O stehen,
mit der Maßgabe, daß wenn $R_7$ für >C=O steht, Y dann für eine Kohlenstoff-Stickstoff-Bindung oder $-CH(R_8)$ steht und $R_8$ für Wasserstoff steht, und q fiir $-CH_2-$ steht,
$R_5$ für Wasserstoff, Niederalkyl, Benzyl oder eine Oxycarbonylgruppe steht, wie es in Anspruch 1 definiert ist, und
$R_6$ für Wasserstoff, Halogen, Hydroxy, Niederalkyl oder Niederalkoxy steht,
und pharmazeutisch annehmbare, nicht-toxische Salze hiervon.

**3.** Verbindung nach Anspruch 1, wobei die Verbindung D,L-1,2,3,4-Tetrahydroisochinolin-1-oyl-L-prolyl-Largininaldehydsulfat ist.

**4.** Verbindung nach Anspruch 1, wobei die Verbindung D-1,2,3,4-Tetrahydroisochinolin-1-oyl-L-prolyl-Largininaldehydsulfat ist.

**5.** Verbindung nach Anspruch 1, wobei die Verbindung D-1,2,3,4-Tetrahydroisochinolin-3-oyl-L-prolyl-Largininaldehyd ist und pharmazeutisch annehmbare Salze hiervon.

**6.** Verbindung nach einem der Ansprüche 1-5 zur Verwendung als antithrombotisches Mittel.

**7.** Pharmazeutische Formulierung, die als Wirkstoff eine Verbindung nach einem der Ansprüche 1-5 zusammen mit einem oder mehreren pharmazeutisch annehmbaren Trägern, Hilfsmitteln oder Verdünnungsmitteln hierfür enthält.

**Patentansprüche für folgende Vertragsstaaten : ES, GR**

**1.** Verfahren zur Herstellung einer Verbindung der Formel

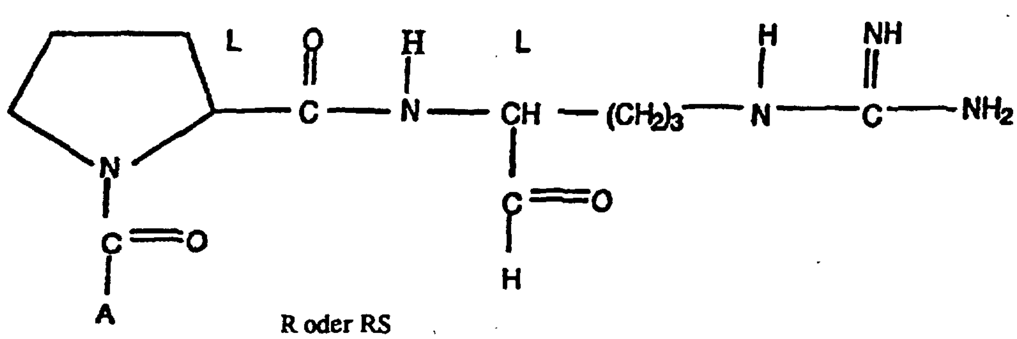

R oder RS

1

worin A fiir eine bicyclische Gruppe der folgenden Formel steht

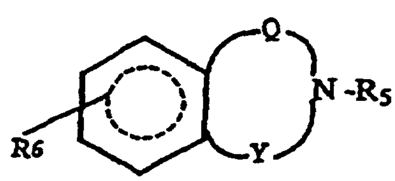

2

worin Q fiir einen Rest mit einem Kohlenstoff steht, der dargestellt wird durch $-CH_2-$ und

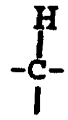

oder einen Rest mit zwei Kohlenstoffen, der dargestellt wird durch $-CH_2-CH_2-$ oder

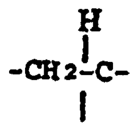

Y für einen Rest mit einem Kohlenstoff steht, der dargestellt wird durch $-CH_2-$ oder

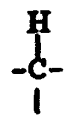

mit der Maßgabe. daß wenn Q für

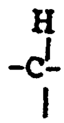 oder 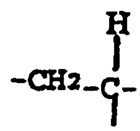

steht. Y dann für $-CH_2-$ steht, und wenn Y für

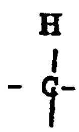

- steht, Q dann für $-CH_2-$ oder $-CH_2-CH_2-$ steht.
$R_5$ für Wasserstoff oder eine Oxycarbonylgruppe der Formel $R_4$-OC(O)- steht,
worin $R_4$ für $C_1$-$C_6$ Alkyl, $C_2$-$C_6$ Alkenyl, $C_3$-$C_7$ Cycloalkyl, Benzyl, Nitrobenzyl, Diphenylmethyl oder eine Phenylgruppe der folgenden Formel steht

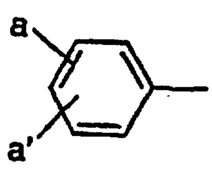

worin a und a' unabhängig fiir Wasserstoff. Niederalkyl, Niederalkoxy, Halogen, Trifluormethyl. Hydroxy. Hydromethyl, Amino oder Aminomethyl stehen und
$R_6$ für Wasserstoff, Halogen, Hydroxy, Niederalkyl oder Niederalkoxy steht und der gestrichelte Ring innerhalb des sechsgliedrigen Rings anzeigt, daß der Ring ein aromatischer Ring ist
und pharmazeutisch annehmbarer, nicht-toxischer Salze hiervon.

**2.** Verfahren zur Herstellung einer Verbindung der Formel

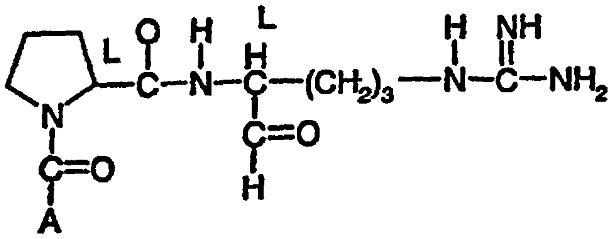

R oder RS

worin A fiir eine bicyclische Gruppe der folgenden Formel steht

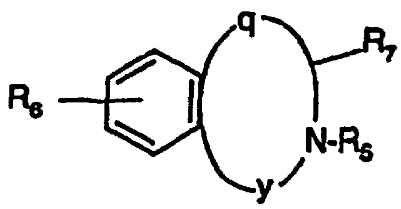 3

worin q für eine Kohlenstoff-Kohlenstoff-Bindung oder $-CH_2-$ steht,
Y für eine Kohlenstoff-Stickstoff-Bindung oder $-CH(R_8)-$ steht und
$R_7$ und $R_8$ für Wasserstoff oder >C=O stehen,
mit der Maßgabe, daß wenn $R_7$ für >C=O steht, Y dann für eine Kohlenstoff-Stickstoff-Bindung oder $-CH(R_8)$ steht und $R_8$ für Wasserstoff steht, und q für $-CH_2-$ steht.
$R_5$ für Wasserstoff. Niederalkyl, Benzyl oder eine Oxycarbonylgruppe steht, wie dies in Anspruch 1 definiert ist. und
$R_6$ für Wasserstoff. Halogen. Hydroxy. Niederalkyl oder Niederalkoxy steht,
und pharmazeutisch annehmbarer, nicht-toxischer Salze hiervon.

**3.** Verfahren nach Anspruch 1 zur Herstellung von D.L-1,2,3,4-Tetrahydroisochinolin-1-oyl-L-prolyl-L-argininaldehydsulfat.

**4.** Verfahren nach Anspruch 1 zur Herstellung von D-1,2,3,4-Tetrahydroisochinolin-1-oyl-L-prolyl-L-argininaldehydsulfat.

**5.** Verfahren nach Anspruch 1 zur Herstellung von D.L-1,2,3,4-Tetrahydroisochinolin-1-oyl-L-prolyl-L-argininaldehydsulfat und pharmazeutisch annehmbarer Salze hiervon.

**Revendications**

**Revendications pour les Etats contractants suivants : AT, BE, CH, DE, DK, FR, GB, IT, LI, LU, NL, SE**

**1.** Composé répondant à la formule

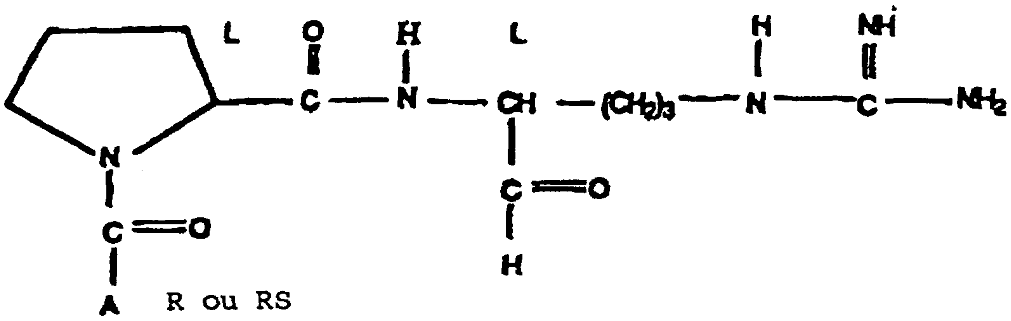

R ou RS

1

dans laquelle A représente un groupe bicyclique répondant à la formule

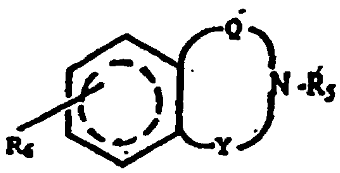

2

dans laquelle Q représente un radical contenant un atome de carbone représenté par un groupe $-CH_2-$ et par un groupe

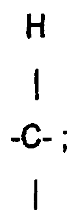

ou représente un radical contenant deux atomes de carbone représenté par un groupe $-CH_2-CH_2-$ ou par un groupe

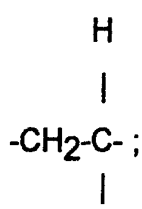

Y représente un radical contenant un atome de carbone représenté par un groupe $-CH_2-$ ou par un groupe

H

|

-C- ;

|

à condition que, lorsque Q représente un groupe

H H

| |

-C- ou un groupe $-CH_2-C$

| |

Y représente un groupe $-CH_2-$, et lorsque Y représente un groupe

H

|

-C-,

|

Q représente un groupe $-CH_2-$ ou un groupe $-CH_2-CH_2-$ ;

$R_5$ représente un atome d'hydrogène ou un groupe oxycarbonyle répondant à la formule $R_4$-O-C(O)- dans laquelle $R_4$ représente un groupe alkyle en $C_1$-$C_6$, un groupe alcényle en $C_2$-$C_6$, un groupe cycloalkyle en $C_3$-$C_7$, un groupe benzyle, un groupe nitrobenzyle, un groupe diphénylméthyle ou un groupe phényle répondant à la formule

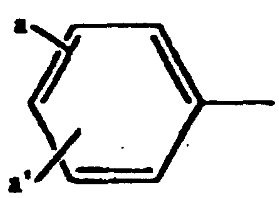

dans laquelle a et a' représentent, indépendamment l'un de l'autre, un atome d'hydrogène, un groupe alkyle inférieur, un groupe alcoxy inférieur, un atome d'halogène, un groupe trifluorométhyle, un groupe hydroxyle, un groupe hydroxyméthyle, un groupe amino ou un groupe aminométhyle; et

$R_6$ représente un atome d'hydrogène, un atome d'halogène, un groupe hydroxyle, un groupe alkyle inférieur ou un groupe alcoxy inférieur ; et le cercle présentant des traits à l'intérieur du noyau hexagonal indique que le noyau est un noyau aromatique ;

et ses sels non toxiques pharmaceutiquement acceptables.

2. Composé répondant à la formule

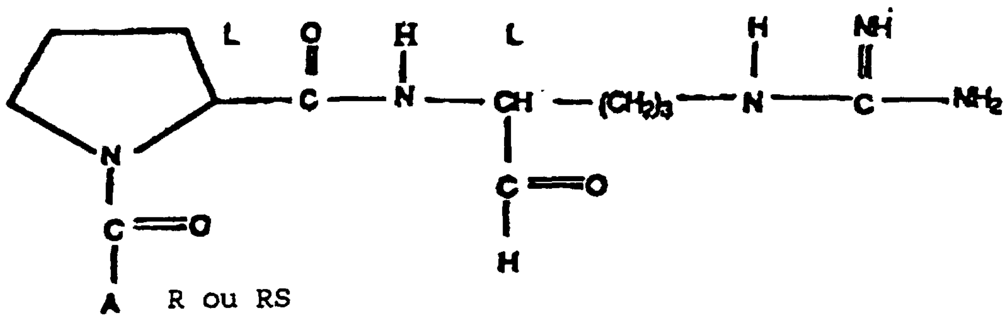

dans laquelle A représente un groupe bicyclique répondant à la formule

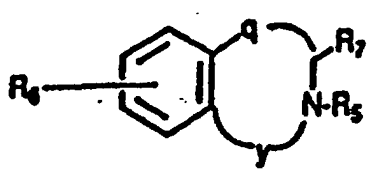

3

dans laquelle q représente une liaison carbone - carbone ou un groupe $-CH_2-$ ; Y représente une liaison carbone - azote ou encore un groupe $-CH(R_8)-$ ; et $R_7$ et $R_8$ représentent un atome d'hydrogène ou un groupe >C=O, à condition que, lorsque $R_7$ représente un groupe >C=O, Y représente une liaison carbone-azote, un groupe -CH $(R_8)$- et $R_8$ représente un atome d'hydrogène et q représente un groupe $-CH_2-$ ; $R_5$ représente un atome d'hydrogène, un groupe alkyle inférieur, un groupe benzyle ou un groupe oxycarbonyle tels que définis à la revendication 1, et $R_6$ représente un atome d'hydrogène, un atome d'halogène, un groupe hydroxyle, un groupe alkyle inférieur ou un groupe alcoxy inférieur ; et ses sels non toxiques pharmaceutiquement acceptables.

3. Composé selon la revendication 1, ledit composé étant le sulfate de l'aldéhyde de la DL-1,2,3,4-tétrahydroisoquinolin-1-oyl-L-prolyl-L-arginine.

4. Composé selon la revendication 1, ledit composé étant le sulfate de l'aldéhyde de la D-1,2,3,4-tétrahydroisoquinolin-1-oyl-L-prolyl-L-arginine.

5. Composé selon la revendication 1, ledit composé étant l'aldéhyde de la D-1,2,3,4-tétrahydroisoquinolin-3-oyl-L-prolyl-L-arginine, ainsi que ses sels pharmaceutiquement acceptables.

6. Composé selon l'une quelconque des revendications 1 à 5 à utiliser à titre d'agent antithrombotique.

7. Formulation pharmaceutique comprenant, à titre d'ingrédient actif, un composé tel que revendiqué dans l'une quelconque des revendications 1 à 5, en association avec un ou plusieurs supports, excipients ou diluants pharmaceutiquement acceptables pour ledit composé.

**Revendications pour les Etats contractants suivants : ES, GR**

1. Procédé pour préparer un composé répondant à la formule

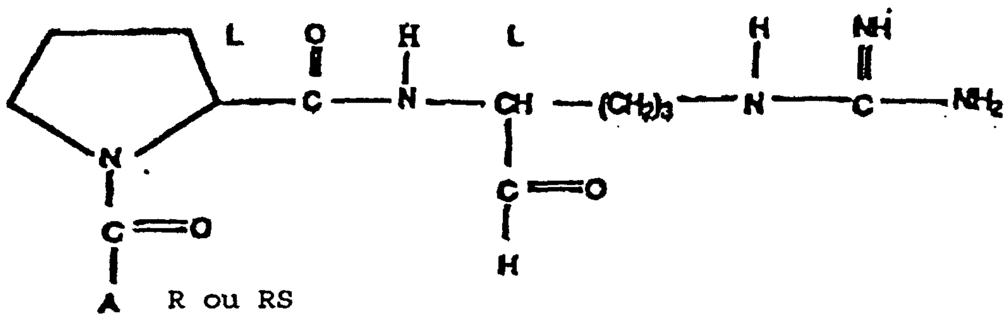

R ou RS

1

dans laquelle A représente un groupe bicyclique répondant à la formule

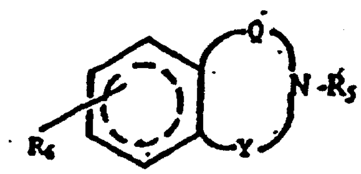

2

dans laquelle Q représente un radical contenant un atome de carbone représenté par un groupe $-CH_2-$ et par un groupe

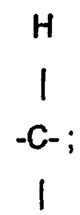

ou représente un radical contenant deux atomes de carbone représenté par un groupe $-CH_2-CH_2-$ ou par un groupe

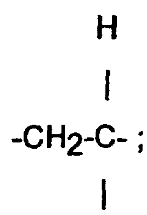

Y représente un radical contenant un atome de carbone représenté par un groupe $-CH_2-$ ou par un groupe

H
|
-C- ;
|

à condition que, lorsque Q représente un groupe

H H
| |
-C- ou un groupe $-CH_2-C$
| |

Y représente un groupe $-CH_2-$, et lorsque Y représente un groupé

H
|
-C-,
|

Q représente un groupe $-CH_2-$ ou un groupe $-CH_2-CH_2-$ ;
$R_5$ représente un atome d'hydrogène ou un groupe oxycarbonyle répondant à la formule $R_4$-O-C(O)- dans laquelle $R_4$ représente un groupe alkyle en $C_1$-$C_6$, un groupe alcényle en $C_2$-$C_6$, un groupe cycloalkyle en $C_3$-$C_7$, un groupe benzyle, un groupe nitrobenzyle, un groupe diphénylméthyle ou un groupe phényle répondant à la formule

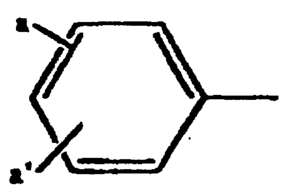

dans laquelle a et a' représentent, indépendamment l'un de l'autre, un atome d'hydrogène, un groupe alkyle inférieur, un groupe alcoxy inférieur, un atome d'halogène, un groupe trifluorométhyle, un groupe hydroxyle, un groupe hydroxyméthyle, un groupe amino ou un groupe aminométhyle; et
$R_6$ représente un atome d'hydrogène, un atome d'halogène, un groupe hydroxyle, un groupe alkyle inférieur ou un groupe alcoxy inférieur ; et le cercle présentant des traits à l'intérieur du noyau hexagonal indique que le noyau est un noyau aromatique ;
et ses sels non toxiques pharmaceutiquement acceptables.

**2.** Procédé pour préparer un composé répondant à la formule

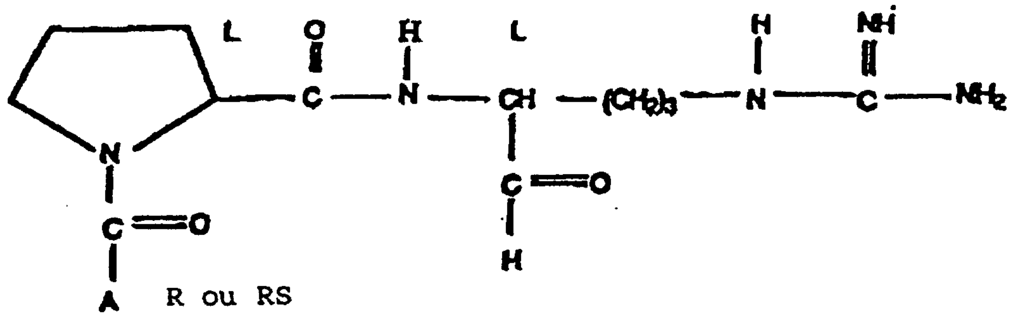

dans laquelle A représente un groupe bicyclique répondant à la formule

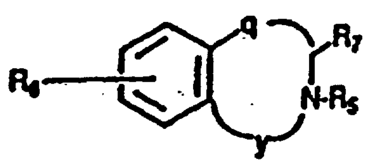

3

dans laquelle q représente une liaison carbone - carbone ou un groupe $-CH_2-$; Y représente une liaison carbone - azote ou encore un groupe $-CH(R_8)-$ ; et $R_7$ et $R_8$ représentent un atome d'hydrogène ou un groupe >C=O, à condition que, lorsque $R_7$ représente un groupe >C=O, Y représente une liaison carbone-azote, un groupe $-CH(R_8)-$ et $R_8$ représente un atome d'hydrogène et q représente un groupe $-CH_2-$; $R_5$ représente un atome d'hydrogène, un groupe alkyle inférieur, un groupe benzyle ou un groupe oxycarbonyle tels que définis à la revendication 1, et $R_6$ représente un atome d'hydrogène, un atome d'halogène, un groupe hydroxyle, un groupe alkyle inférieur ou un groupe alcoxy inférieur ; et ses sels non toxiques pharmaceutiquement acceptables.

**3.** Procédé selon la revendication 1, pour la préparation du sulfate de l'aldéhyde de la DL-1,2,3,4-tétrahydroisoquinolin-1-oyl-L-prolyl-L-arginine.

**4.** Procédé selon la revendication 1, pour la préparation du sulfate de l'aldéhyde de la D-1,2,3,4-tétrahydroisoquinolin-1-oyl-L-prolyl-L-arginine.

**5.** Procédé selon la revendication 1, pour la préparation du sulfate de l'aldéhyde de la D-1,2,3,4-tétrahydroisoquinolin-3-oyl-L-prolyl-L-arginine et de ses sels pharmaceutiquement acceptables.